(19) Europäisches Patentamt
European Patent Office
Office européen des brevets

(11) Veröffentlichungsnummer: **0 071 901**
**A1**

# (12) EUROPÄISCHE PATENTANMELDUNG

(21) Anmeldenummer: **82106881.4**

(51) Int. Cl.³: **C 07 C 115/00**

(22) Anmeldetag: **30.07.82**

(30) Priorität: **06.08.81 DE 3131115**

(43) Veröffentlichungstag der Anmeldung: **16.02.83**
**Patentblatt 83/7**

(84) Benannte Vertragsstaaten: **BE CH DE FR GB IT LI NL**

(71) Anmelder: **HOECHST AKTIENGESELLSCHAFT,**
**Postfach 80 03 20, D-6230 Frankfurt/Main 80 (DE)**

(72) Erfinder: **Siegel, Herbert, Dr., Am Schieferberg 10,**
**D-6233 Kelkheim (Taunus) (DE)**

## (54) Verfahren zur Herstellung von 1.3-Diaryltriazenen.

(57) 1.3-Diaryltriazene werden hergestellt durch Umsetzung von gasförmigen Alkylnitriten mit primären aromatischen Monoaminen in – vorzugsweise ein polares protisches Lösungsmittel enthaltender – flüssiger Phase in Gegenwart einer geringen Menge einer Säure.

Die Reaktionsprodukte sind teils Endprodukte, z.B. Schäumer bei der Kunststoffherstellung, teils Zwischenprodukte auf verschiedenen Sachgebieten wie dem Polymeren-, Farbstoff- und Pharmasektor.

EP 0 071 901 A1

Verfahren zur Herstellung von 1.3-Diaryltriazenen

1.3-Diaryltriazene sind Verbindungen der allgemeinen Formel

Ar-N=N-NH-Ar,

worin Ar = gegebenenfalls substituierter Arylrest.

Sie finden Verwendung als solche z.B. als Schäumer bei der Kunststoffherstellung, hauptsächlich jedoch als Zwischenprodukte vor allem zur Herstellung von aromatischen Diaminen sowie von Arylhydrazinen (insbesondere Phenylhydrazin), die ihrerseits wieder auf bekannte Weise zu entsprechenden Endprodukten vorwiegend auf dem Polymeren-, Farbstoff- und Pharmasektor weiterverarbeitet werden.

Durch Erhitzen und/oder die Einwirkung von Säuren lagern sich die 1.3-Diaryltriazene zu den jeweiligen Aminoazoverbindungen um. Nach dem Verfahren der EP-OS 13 643 beispielsweise wird diese Umlagerung mittels etwa 0.4 bis 2.0 Gew.-%, bezogen auf die Triazen-haltige Lösung, einer starken Säure - insbesondere Salpetersäure - durchgeführt.

Für die Herstellung der 1.3-Diaryltriazene ist eine Reihe verschiedener Methoden bekannt. Eine solche Methode beschreiben z.B. G.Vernin et al. in Synthesis 1977, S. 691 - 693. Die Methode besteht in der Umsetzung von i-Amylnitrit mit primären aromatischen Monoaminen bei Raumtemperatur in unpolaren aprotischen Lösungsmitteln (wie Hexan, Petrolether etc.). Dabei sollen in kurzen Reaktionszeiten hohe Triazenausbeuten erhalten werden.

Nach dem einzigen Beispiel für die Herstellung eines solchen Triazens [1.3-Bis-(3.5-dichlorphenyl)-triazen: Umsetzung von i-Amylnitrit mit 3.5-Dichloranilin bei Raumtemperatur in Petrolether/Benzol im Labormaßstab] soll in 30 Minuten

eine Ausbeute von 90 % an dem gewünschten Produkt erhalten werden. Dabei wurde allerdings das i-Amylnitrit in erheblichem (3-fachem) Überschuß eingesetzt. Die Aufarbeitung des Reaktionsansatzes geschah durch Eindampfen im Rotationsverdampfer, Absaugen des verbliebenen Feststoffes, Auswaschen und Trocknen.

Für die Durchführung in technischem Maßstab ist diese Arbeitsweise jedoch hauptsächlich wegen des eingesetzten i-Amylnitrit-Überschusses und dem Eindampfen des Reaktionsansatzes im Rotationsverdampfer kaum vorteilhaft. In der Literaturstelle ist nichts darüber ausgesagt, ob das Eindampfen im Vakuum oder bei Normaldruck durchgeführt wurde. Da als Vakuum in solchen Fällen üblicherweise Wasserstrahlvakuum verwendet wird, ist es hier - ohne besondere und u. U. oft recht aufwendige Vorkehrungen - kaum zu vermeiden, daß ein Teil der ziemlich leicht flüchtigen Lösungsmittel mit in den Wasserstrom und damit ins Abwasser gelangt, was aus Umweltschutzgründen jedenfalls bei größeren Ansätzen zu vermeiden ist. Das Eindampfen im Vakuum könnte aber wenigstens ohne Temperaturerhöhung erfolgen. Letzteres - eine Temperaturerhöhung - ist beim Eindampfen bei Normaldruck notwendig. Für manche - infolge bestimmter Substituenten - stabilere 1.3-Diaryltriazene ist dies sicher kaum schädlich; andere - und dies ist die überwiegende Mehrzahl der normalen 1.3-Diaryltriazene - werden wegen ihrer relativen Instabilität jedoch bei erhöhter Temperatur leicht umgelagert und/oder anderweitig zersetzt, was einen erheblichen Nachteil darstellt.

Nach G. Vernin et al. ist die Umsetzung von i-Amylnitrit mit primären aromatischen Monoaminen bei Raumtemperatur auch in polaren protischen Lösungsmitteln (Methanol!) durchführbar, wenn noch ein Quecksilbersalz wie z.B. $Hg(OCOCH_3)_2$ zugesetzt wird. Man erhält dann allerdings nicht das freie 1.3-Triazen, sondern einen entsprechenden Hg-Komplex. Über

die Freisetzung des Triazens aus dem Komplex ist in der Literaturstelle nichts ausgesagt.

Ohne den Zusatz eines Quecksilbersalzes erfolgt die Umsetzung zwischen Alkylnitrit und primärem aromatischen Monoamin bei Raumtemperatur in Methanol - wie eigene Versuche ergeben haben - erst nach einer erheblichen (mehrstündigen) Induktionszeit.

In dem Bestreben, das von G.Vernin et al. publizierte Verfahren in einer auch für technische Verhältnisse vorteilhaften Weise zu verbessern, wurde nun gefunden, daß dieses Ziel haupsächlich durch die Verwendung gasförmiger Alkylnitrite sowie den Zusatz einer geringen Menge Säure zu dem - vorteilhaft in einem polaren protischen Lösungsmittel gelösten - primären aromatischen Monoamin erreicht wird.

Erfindungsgegenstand ist daher ein Verfahren zur Herstellung von 1.3-Diaryltriazenen durch Umsetzung von Alkylnitriten mit primären aromatischen Monoaminen bei Raum- oder nur wenig darüber erhöhter Temperatur,
das dadurch gekennzeichnet ist, daß man bei der angewandten Temperatur gasförmige Alkylnitrite in bei dieser Temperatur flüssige oder in einem polaren protischen Lösungsmittel gelöste primäre aromatische Monoamine
in Gegenwart einer geringen Menge Säure einleitet.

Nach dem Verfahren werden hohe Triazen-Ausbeuten erhalten. Ein Alkylnitrit-Überschuß ist nicht erforderlich und etwaige Induktionszeiten (wie bei der ohne Säurezusatz durchgeführten Umsetzung) treten nicht auf. Wegen des hydrophilen Charakters der polaren protischen Lösungsmittel - insbesondere der hier vorzugsweise verwendeten niederen Alkohole - ist die Aufarbeitung des Reaktionsansatzes durch Ausfällung der Triazene mit Wasser möglich. Dadurch gelangt kein Lösungsmittel (wie etwa beim Abziehen des Lösungsmittels im Wasserstrahlvakuum bei dem Verfahren von G. Vernin et al.) ins Abwasser und die Reaktionsprodukte werden auch keinen Temperaturbelastungen (wie bei dem Verfahren von G. Vernin, wenn das Lösungsmittel dort bei Normaldruck und - dadurch bedingt - bei erhöhter Temperatur abgezogen wird) ausgesetzt. Das Verfahren eignet sich daher für die Durchführung in technischem Maßstab in ausgezeichneter Weise.

Es war außerordentlich überraschend, daß die Gegenwart bzw. der Zusatz einer geringen Menge Säure die gewünschte Umsetzung katalysiert, ohne gleichzeitig auch die bekanntermaßen säurekatalysierte Umlagerung der 1.3-Diaryltriazene zu den entsprechenden p-Aminoazobenzolen in einem unerwünscht hohen Ausmaß zu bewirken. Wahrscheinlich sind für diesen überraschenden Reaktionsverlauf die anderen Bedingungen des erfindungsgemäßen Verfahrens (Verwendung gasförmiger Alkylnitrite, vorzugsweise Einsatz von polaren protischen Lösungsmitteln) zumindest mit verantwortlich.

Als bei Raum- oder nur wenig darüber erhöhter Temperatur gasförmige Alkylnitrite kommen vorzugsweise Methylnitrit $CH_3NO_2$ und/oder Ethylnitrit $C_2H_5NO_2$ infrage. Diese können z.B. nach dem in der DE-PS 1 156 775 beschriebenen Verfahren aus $N_2O_3$ bzw. nitrosen Gasen (= Gemische aus hauptsächlich NO und $NO_2$) und Alkoholen erhalten werden. Es wird dabei vorteilhaft so verfahren, daß ein Gemisch aus NO, $NO_2$ und Stickstoff in den Alkohol, vorzugsweise Methanol oder Ethanol, in einem entsprechenden Reaktor (in welchem eine gute Durchmischung von Gas und Flüssigkeit stattfindet) eingegast wird. Das $NO/NO_2$-Molverhältnis im Nitrosegas-Gemisch kann zwischen etwa 100:1 und etwa 40:60 liegen, beträgt jedoch vorteilhaft etwa 1:1; der Inertgas-Anteil kann zwischen 0 und etwa 99 % schwanken. Ein derartiges Gasgemisch läßt sich leicht durch Oxidation von NO mit Luft oder auch reinem Sauerstoff erhalten. Es kann ohne weiteres auch ein technisches Nitrosegemisch, wie es bei der Salpetersäureproduktion anfällt, verwendet werden.

Der Alkylnitrit-Reaktor wird bei einer Temperatur betrieben, die so gewählt ist, daß das gebildete Alkylnitrit gasförmig aus dem Reaktionsgemisch ausgetragen wird. Das Methyl- und Ethylnitrit (Kp = -12°C bzw. 16°C) kann sogar ohne weiteren Aufwand bei Normaltemperatur vom Inertgasstrom ausgetragen werden, wobei der Gasstrom zweckmäßigerweise einen Kühler passiert, um das Mitreißen von alzu viel Alkohol zu vermeiden. Die Menge des Alkylnitrits wird so einfach durch die eingegaste Nitrosegasmenge bestimmt. In einer bevorzugten Ausführungsform der Erfindung wird dann das so hergestellte Alkylnitrit unmittelbar in einer an den Alkylnitrit-Reaktor angeschlossenen weiteren Stufe in das primäre aromatische Monoamin eingeleitet, welches dort als solches in flüssiger oder - vorzugsweise - in einem polaren protischen Lösungsmittel gelöster Form vorliegt und noch eine geringe Säuremenge enthält.

Als primäre aromatische Monoamine kommen im Prinzip alle möglichen solche Amine in Frage, wobei die bei Raum- oder nur wenig darüber erhöhter Temperatur flüssigen Amine als solche oder in gelöster Form, die festen Amine nur in gelöster Form, eingesetzt werden. Bevorzugt sind die unter die nachstehende allgemeine Formel fallenden primären aromatischen Monoamine:

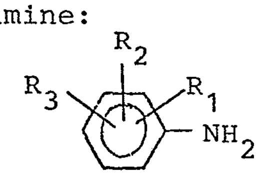

worin $R_1$, $R_2$ und $R_3$ = unabhängig voneinander H, Halogen (F, Cl, Br, J), Alkyl (vorzugsweise ($C_1$-$C_4$-Alkyl) und $NO_2$, insbesondere nur H.

Beispielhafte derartige Monoamine sind:

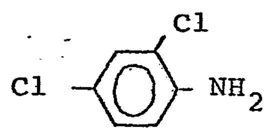

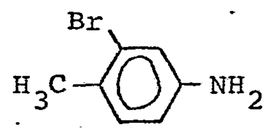

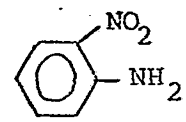

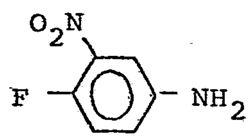

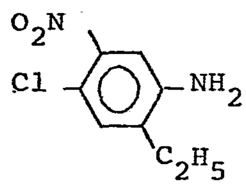

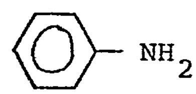

etc.

Die Reaktionstemperatur kann im allgemeinen zwischen etwa 10 und 50°C liegen; vorzugsweise liegt sie zwischen etwa 20 und 35°C.

Als polare protische Lösungsmittel kommen im Prinzip alle unter diesen Begriff fallende Flüssigkeiten in Frage, welche bei den Reaktionsbedingungen nicht selbst reagieren. Bevorzugte polare protische Lösungsmittel sind die $C_1$-$C_4$-Alkohole, insbesondere die dem verwendeten Alkylnitrit zugrundeliegenden Alkohle, also in erster Linie Methanol und Ethanol.

Das Verhältnis von polarem protischen Lösungsmittel und primärem aromatischen Monoamin kann in weiten Grenzen schwanken. Allgemein gängige Grenzen sind etwa 5 : 95 bis etwa 90 : 10 (Gewichtsverhältnis polare protische Lösungsmittel : Amin).

Als den Aminen zugesetzte bzw. zuzusetzende Säuren können praktisch alle möglichen organischen und anorganischen, schwachen und starken Säuren verwendet werden. Bevorzugt ist

der Einsatz von Mineralsäuren, insbesondere von Salzsäure.

Die Säuremenge kann normalerweise zwischen etwa 0,01 und 5, vorzugsweise zwischen etwa 0,05 und 1 Äquivalent-% pro Mol primäres aromatisches Monoamin betragen. Dies bedeutet etwa 0,01 bis 5, vorzugsweise etwa 0,05 bis 1 Mol-% einer einbasischen Säure, die Häfte dieser Menge einer zweibasischen Säure, ein Drittel einer dreibasischen Säure etc., jeweils pro Mol primäres aromatisches Monoamin. Bei höheren Säurezusätzen erfolgt dann in zunehmendem Maß die - unerwünschte - Umlagerung der zunächst gebildeten 1.3-Triazene hauptsächlich zu den entsprechenden p-Aminoazobenzolen.

Bei dem Verfahren wird das gewünschte Triazen meist in Form einer Kristallsuspension erhalten. Diese kann in bestimmten Fällen ohne Isolierung des Triazens weiter verwendet werden, z.B. wenn die Reduktion zu Phenylhydrazin oder die Umlagerung zu p-Phenylendiamin angestrebt wird.

Wenn die Isolierung der Triazene beabsichtigt ist, geschieht dies zweckmäßig durch Absaugen oder -drücken der Produktlösung aus dem Reaktor und Zugabe von Wasser. Man erhält die Triazene dann als gelbe bis bräunlich gefärbte Kristalle, die abgesaugt werden können. Einer besonderen Temperaturbelastung unterliegen die Triazene bei dieser Isolierungsmethode (welche bei dem Verfahren von G. Vernin a.a.O. wegen der dortigen Verwendung mit Wasser nicht mischbarer unpolarer aprotischer Lösungsmittel nicht anwendbar ist) nicht. Die Aufarbeitung der Mutterlauge und des Filtrats kann dann an einer von der Triazengewinnung unabhängigen Stelle in nicht umweltbelastender Weise geschehen.

Die nach dem erfindungsgemäßen Verfahren erzielten Ausbeuten liegen in der Größenordnung der auch von G. Vernin a.a.O. nach dessen Verfahrensweise erhaltenen Ausbeuten. Die Geschwindigkeit der Triazenbildung hängt natürlich in z.T. erheblichem Maß von der Art der Substituenten am aromatischen Kern der Ausgangsverbindungen ab. Elektronen-ziehende Substituenten verlangsamen die Reaktion, während Elektronenschiebende sich beschleunigend auswirken.

Wegen der einfachen, produkt- und umweltschonenden Durchführbarkeit des Verfahrens auch in technischem Maßstab stellt die Erfindung einen erheblichen Fortschritt auf diesem Gebiet dar.

Die Erfindung wird nun durch die nachfolgenden Beispiele näher erläutert. Die Beispiele wurden z.T. nicht bis zum vollständigen Umsatz des eingebrachten primären aromatischen Monoamins geführt. Nach den Erfindungsbeispielen folgt dann noch ein Vergleichsbeispiel, welches zeigt, daß zwischen gasförmigem Alkylnitrit (Methylnitrit) und primärem aromatischen Monoamin (Anilin) in Gegenwart eines polaren protischen Lösungsmittels (Methanol), jedoch in Abwesenheit von Säure, jedenfalls innerhalb von 2 1/2 Stunden praktisch keine Reaktion eintritt.

Beispiel 1

Ein aus 2,7 l/h NO und 3,6 l/h Luft erhaltenes $NO/NO_2$-Gemisch (1:1) wurde bei Raumtemperatur durch Methanol geleitet, und in Methylnitrit umgewandelt. Das entstandene Methylnitrit wurde kontinuierlich in eine Mischung aus 100 g Anilin, 0,25 g (0,25 %) Anilin-Hydrochlorid und 5 ml Methanol geleitet. Die Temperatur des Reaktionsgemisches erhöhte sich während der Umsetzung auf 33 - 34°C, und nach 95 Minuten fiel festes Triazen aus. Nach 110 Minuten wurde der Gasstrom abgestellt und noch 1,5 Stunden nachgerührt. 2,5 g Methylnitrit hatten in dieser Zeit die Lösung unumgesetzt passiert und wurden in einer Kühlfalle bei -78°C aufgefangen. Das Reaktionsgemisch wurde auf 500 ml Wasser gegeben, die Kristalle abgesaugt und getrocknet. Man erhielt 35,1 g hellgelbes Triazen (99,3 % bezogen auf NO; 33,3 % bezogen auf Anilin) GC-Analyse:

0,32 % Anilin
98,47 % 1,3-Diphenyltriazen
0,78 % 4-Aminoazobenzol
0,43 % 5 unbekannte Komponenten

Beispiel 2

Analoges Vorgehen wie in Beispiel 1, jedoch Verwendung von 1 % Anilin-Hydrochlorid als Katalysator lieferte 34,3 g 1,3-Diphenyltriazen (97,0 % bez. auf NO, 32,4 % bez. auf Anilin).

GC-Analyse

2,39 % Anilin
0,16 % 2-Amino-Biphenyl
0,30 % N,N-Diphenylamin
0,19 % a-Amino-Biphenyl
92,92 % 1,3-Diphenyltriazen
2,70 % 4-Aminoazobenzol
1,34 % 8 unbekannte Komponenten

Beispiel 3

Wie in Beispiel 1 beschrieben, wurden zu einer Mischung aus 80 g Anilin, 4 g Anilin-Hydrochlorid (5 % bez. auf Anilin) und 20 ml Methanol ein Strom von Methylnitrit geleitet. Es ergaben sich 33,8 g Produkt (95,5 % bez. auf NO, 31,8 % bez. auf Anilin).

GC-Analyse:

0,18 % 2-Amino-Biphenyl
0,44 % N,N-Diphenylamin
0,22 % 4-Amino-Biphenyl
79,62 % 1,3-Diphenyltriazen

16,97 % 4-Aminoazobenzol
2,57 % 10 unbekannte Komponenten

Beispiel 4

Bei analogem Vorgehen wie in Beispiel 1, jedoch unter Verwendung von Ethanol anstatt Methanol wurde kein Austrag von Ethylnitrit beobachtet und man erhielt 42,0 g 1,3-Diphenyltriazen (97 % bez. auf NO; 39,5 % bez. auf Anilin).
GC-Analyse:

0,1 % Anilin
96,7 % 1,3-Diphenyltriazen
1,8 % 4-Aminoazobenzol
1,4 % 10 unbekannte Komponenten

Beispiel 5

Man ging wie in Beispiel 1 beschrieben vor, verwendete jedoch Isopropanol anstatt Methanol und heizte den ersten Reaktor auf 55°C zum Abdestillieren des Salpetrigsäureesters. Man erhielt 41,2 g 1,3-Diphenyltriazen (94,5 % bez. auf NO; 38,7 % bez. auf Anilin).
GC-Analyse:

0,1 % Anilin
98,0 % 1,3-Diphenyltriazen
0,2 % 4-Aminoazobenzol
1,7 % 13 unbekannte Komponenten

Beispiel 6

Analog Beispiel 1 wurde in einem Umwälzreaktor von 2 l Inhalt zu einem Gemisch aus 1000 g Anilin, 1 g Anilin-Hydrochlorid (= 0,1 % bezogen auf Anilin) und 50 ml Methanol Methylnitrit (aus 8,1 l/h NO; 10,8 l/h Luft und Methanol)

geleitet. Der Reaktorinhalt nahm eine Temperatur von 33 - 35°C an und nach 3 Stunden fiel festes Triazen aus. Die Hälfte des Reaktionsgemisches wurde in einen entsprechenden Reaktor gleicher Größe überführt und der Inhalt beider Reaktoren mit je 1 Liter Methanol verdünnt, daß ein gut rührbares Gemisch entstand. In beide Reaktoren wurde weitere 3,5 h Methylnitrit (aus 8,1 l/h NO; 10,8 l/h Luft und Methanol) geleitet. Die resultierende gelbe Suspension wurde 2 h weitergerührt und dann mit Vakuum aus der Apparatur gezogen und in einer Vorlage mit Wasser das Triazen ausgefällt. Es ergaben sich 710 g (99,5 % bezogen auf NO; 67,2 % bezogen auf Anilin) hellgelbes 1,3-Diphenyltriazen.
GC-Analyse:

0,11 % Anilin
0,04 % 4-Aminobiphenyl
99,54 % 1,3-Diphenyltriazen
0,02 % 4-Aminoazobenzol
0,25 % 1 unbekannte Komponente

<u>Beispiel 7</u>

Analog Beispiel 6 wurde zu 800 g Anilin, 1 g Anilin-Hydrochlorid und 50 ml Methanol 3,5 h lang Methylnitrit geleitet (aus 8,1 l/h NO; 10,8 l/h Luft und Methanol). Nach Halbierung und Verdünnung wurde in beide Reaktoren je 4 h lang Methylnitrit eingegast und über Nacht ausreagieren gelassen. Man erhielt 820 g (100 % bezogen auf NO; 96,6 % bez. auf Anilin) gelbes Triazen.
GC-Analyse:
0,71 % Anilin
0,38 % N,N-Diphenylamin
0,09 % 4-Aminobiphenyl
93,56 % 1,3-Diphenyltriazen
4,27 % 4-Aminoazobenzol
0,98 % 6 unbekannte Komponenten.

Beispiel 8

Analog Beispiel 6 wurde zu 500 g Anilin, 0,5 g Anilin-Hydrochlorid und 20 ml Methanol ein Strom von Methylnitrit geleitet. Das Methylnitrit wurde durch Durchleiten eines technischen Nitrosegemisches (11 % Nitrose, Oxidationsgrad 33 %,38 l/h) durch Methanol erzeugt. Nach 6 h wurde der Gasstrom abgestellt, eine Stunde nachgerührt, das Triazen mit Wasser ausgefüllt, abgesaugt und getrocknet. Man erhielt 148 g hellgelbe Kristalle (99 % bez. auf $N_2O_3$; 28 % bez. auf Anilin).
GC-Analyse:

0,04 % 4-Aminobiphenyl
98,83 % 1,3-Diphenyltriazen
0,78 % 4-Aminoazobenzol
0,33 % 3 unbekannte Komponenten

Beispiel 9

Nach dem in Beispiel 6 beschriebenen Vorgehen wurden substituierte 1,3-Diaryltriazene hergestellt (Tabelle). Es wurde jeweils eine möglichst konzentrierte Lösung des Anilinderivates in Methanol hergestellt, die 0,1 % des jeweiligen Hydrochlorids enthielt. Dazu wurde der halbmolare Anteil (bez. auf Anilinkomponente) Methylnitrit eingegast und über Nacht gerührt, mit Wasser die festen Produkte ausgefällt, abgesaugt und getrocknet.

| Anilin-derivate | Triazenderivate | Ausb. bezogen auf aromat. Amin | Fp. |
| --- | --- | --- | --- |
| 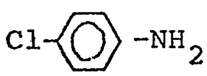 | 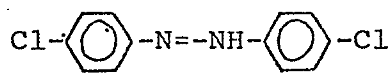 | 94 % | 132°C |
| 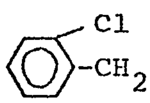 | 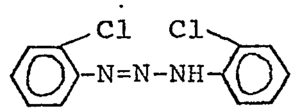 | 87 % | 86°C |
| 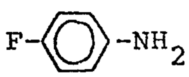 | 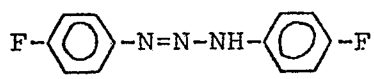 | 67 % | 116°C |
| 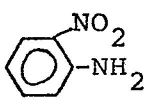 | 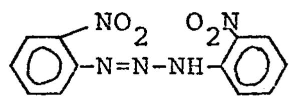 | 60 % | 166°C (Zers.) |
| 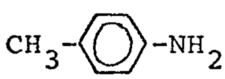 | 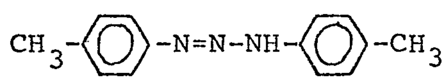 | 67 % | 119°C |
| 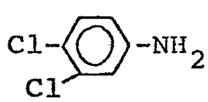 | 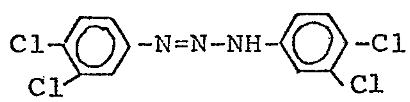 | 65 % | 154°C |
| 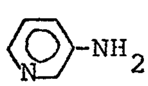 | 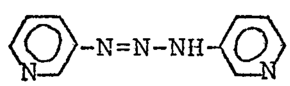 | 54 % | 180°C |

Vergleichsbeispiel

Analog Beispiel 1 wurde Methylnitrit (aus 2,7 l/h NO und 3,6 l/h Luft und Methanol) in 100 g Anilin und 5 ml Methanol geleitet. Die Temperatur des Reaktionsgemisches blieb bei 22°C und in einer nachgeschalteten Falle sammelten sich im Verlauf von 2 1/2 Stunden 17 g Methylnitrit. Gaschromatographische Analyse des Reaktionsgemisches nach dieser Zeit ergab, daß sich weniger als 0,2 % 1,3-Diphenyltriazen gebildet hatten.

1. Verfahren zur Herstellung von 1.3-Diaryltriazenen durch Umsetzung von Alkylnitriten mit primären aromatischen Monoaminen bei Raum- oder nur wenig darüber erhöhter Temperatur,
dadurch gekennzeichnet, daß man bei der angewandten Temperatur gasförmige Alkylnitrite in bei dieser Temperatur flüssige oder in einem polaren protischen Lösungsmittel gelöste primäre aromatische Monoamine in Gegenwart einer geringen Menge Säure einleitet.

2. Verfahren nach Anspruch 1, dadurch gekennzeichnet, daß man als Alkylnitrite Methyl- und/oder Ethylnitrit verwendet.

3. Verfahren nach einem der Ansprüche 1 bis 2, dadurch gekennzeichnet, daß man als primäre aromatische Monoamine Verbindungen der Formel

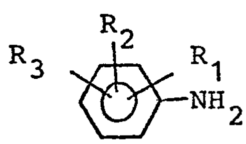

worin $R_1$, $R_2$ und $R_3$ = unabhängig voneinander H, Halogen (F, Cl, Br, J), Alkyl (vorzugsweise $C_1$-$C_4$-Alkyl) und $NO_2$

verwendet.

4. Verfahren nach einem der Ansprüche 1 bis 3, dadurch gekennzeichnet, daß man die Umsetzung bei Temperaturen von etwa 10 bis 50°C, vorzugsweise von etwa 20 bis 35°C, durchführt.

5. Verfahren nach einem der Ansprüche 1 bis 4, dadurch gekennzeichnet, daß man als polare protische Lösungsmittel

$C_1$-$C_4$-Alkohole, insbesondere die den angewandten Alkylnitriten zugrundeliegenden Alkohole, verwendet.

6. Verfahren nach einem der Ansprüche 1 bis 5, dadurch gekennzeichnet, daß man als Säure eine Mineralsäure verwendet.

7. Verfahren nach einem der Ansprüche 1 bis 6, dadurch gekennzeichnet, daß die Säuremenge etwa 0,01 bis 5, vorzugsweise etwa 0,05 bis 1 Äquivalent-% pro Mol primäres aromatisches Monoamin beträgt.

0071901

Europäisches Patentamt

# EUROPÄISCHER RECHERCHENBERICHT

Nummer der Anmeldung

EP 82106881.4

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D,A | SYNTHESIS, Georg Thieme Verlag, Stuttgart, 1977<br>G. VERNIN et al. "Synthesis of 1,3-Diaryltriazenes and their Derivatives by Aprotic Decomposition of Arylamines"<br>Seiten 691-693<br>-- | 1,3,4 |
| D,A | EP - A1 - 0 013 643 (E.I. DU PONT)<br>* Seite 8, Zeilen 16-30; Beispiele; Anspruch 1 *<br>-- | 1,3,4<br>6,7 |
| X | US - A - 2 521 095 (HENRY H. RICHMOND)<br>* Beispiele III,IV; Ansprüche *<br>-- | 1,2,4 |
| A | DE - B2 - 2 453 216 (HOECHST)<br>* Patentanspruch *<br>---- | 1,3,4-7 |

KLASSIFIKATION DER ANMELDUNG (Int. Cl.³)

C 07 C 115/00

RECHERCHIERTE SACHGEBIETE (Int Cl.³)

C 07 C 107/00
C 07 C 115/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 14-10-1982 | REIF |